# EUROPÄISCHE PATENTSCHRIFT

(11) **EP 0 776 657 B1**
(45) Veröffentlichungstag und Bekanntmachung des Hinweises auf die Patenterteilung: **26.03.2003**
(21) Anmeldenummer: 96117640.1
(22) Anmeldetag: 04.11.1996
(51) Int. Cl.: A61K 7/50, A61K 7/48

(54) **Kosmetische und/oder pharmazeutische Emulsionen**
Cosmetic and/or pharmaceutical emulsions
Emulsions cosmétiques et/ou pharmaceutiques

(30) Priorität: 11.11.1995 DE 19542139
(43) Veröffentlichungstag der Anmeldung: 04.06.1997
(73) Patentinhaber: Cognis Deutschland GmbH & Co. KG, 40589 Düsseldorf (DE)
(72) Erfinder: Ansmann, Achim, Dr., 40699 Erkrath (DE); Stoll, Gerhard, Dr., 41352 Korschenbroich (DE); Fabry, Bernd, Dr., 41352 Korschenbroich (DE)

(56) Entgegenhaltungen:
- EP-A- 0 366 070
- WO-A-92/06778
- WO-A-92/07543
- WO-A-94/01076
- WO-A-94/03150
- WO-A-94/07458
- WO-A-95/13863
- DE-A- 4 422 404
- DE-A- 19 524 121
- DATABASE WPI Week 9250 Derwent Publications Ltd., London, GB; AN 92-411555 XP002016634 & JP 04 308 524 A (KAO CORP.) , 30.Oktober 1992

## Beschreibung

### Gebiet der Erfindung

Die Erfindung betrifft kosmetische und pharmazeutische Emulsionen mit verbesserter Stabilität, enthaltend Alkyloligoglucoside, Fettalkohole und kationische Polymere in ausgewählten Mischungsverhältnissen.

### Stand der Technik

Aus der Europäischen Patentschrift **EP-B1 0 553 241** (SEPPIC) ist die Verwendung von Mischungen aus Alkyloligoglucosiden, Fettalkoholen und gegebenenfalls Polyglucose zur Herstellung von Emulsionen bekannt. Auch gemäß der Lehre der internationalen Patentanmeldung **WO 92/07543** (Henkel) lassen sich Alkyloligoglucoside zusammen mit Fettalkoholen und Partialglyceriden als kosmetische Emulgatoren einsetzen.

Zur Herstellung kosmetischer und pharmazeutischer Mittel, wie beispielsweise Cremes, Lotionen oder Salben, werden neben Ölkörpern in vielen Fällen auch Tenside eingesetzt. Es zeigt sich dabei, daß die resultierenden Emulsionen zwar bei Raumtemperatur stabil sind und eine ausreichend hohe Viskosität aufweisen, die jedoch bei längerer Lagerung, zumal bei Temperaturbelastung, allmählich zusammenbricht. Es entstehen dünnflüssige Produkte, in einer Reihe von Fällen kann es auch zu Entmischungen kommen.

Die Aufgabe der Erfindung hat somit darin bestanden, Emulsionen für kosmetische oder pharmazeutische Anwendungen auf Basis von Alkyloligoglucosid/Fettalkoholmischungen zur Verfügung zu stellen, die frei von den geschilderten Nachteilen des Stands der Technik sind, d.h. die auch bei Temperaturbelastung über längere Zeit lagerstabil sind.

### Beschreibung der Erfindung

Gegenstand der Erfindung sind kosmetische und/oder pharmazeutische Emulsionen, enthaltend - bezogen auf den Emulgatoranteil -
(a) mindestens 10 Gew.-% C₁₆-C₂₂-Alkyloligoglucoside,
(b) mindestens 50 Gew.-% C₁₆-C₂₂-Fettalkohole und
(c) 0,1 bis 10 Gew.-% kationische Polymere,
mit der Maßgabe, daß sich die Mengenangaben zu 100 Gew.-% ergänzen.

Überraschenderweise wurde gefunden, daß Mischungen von Alkyloligoglucosiden und Fettalkoholen innerhalb definierter Einsatzverhältnisse zusammen mit Kationpolymeren Emulsionen ergeben, die lagerstabil sind, d.h. deren Viskosität sich auch bei längerer Lagerung bei erhöhten Temperaturen nicht verändert. Die Erfindung schließt die Erkenntnis ein, daß die Auswahl der Zusatzstoffe für die Stabilität der Emulsionen entscheidend ist, da mit anionischen, nichtionischen und kationischen Tensiden nur Emulsionen einer wesentlich geringeren Stabilität erhalten werden.

### Alkyloligoglucoside

In einer bevorzugten Ausführungsform sind Alkyloligoglucoside der Formel **(I)** enthalten,

**R**^{**1**}**O-[G]**_{**p**} **(I)**

in der R¹ für einen linearen, gesättigten Alkylrest mit 16 bis 22 Kohlenstoffatomen, G für einen Glucoserest und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1-0 301 298** und **WO 90/03977** verwiesen. Die Indexzahl p in der allgemeinen Formel **(I)** gibt den Oligomerisierungsgrad (DP), d. h. die Verteilung von Monound Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglucosid eine analytisch ermittelte rechnerische Größe, die meistens eine gebrochene Zahl darstellt. Vorzugsweise werden Alkyloligoglucoside mit einem mittleren Oligomerisierungsgrad p von 1,1 bis 3,0 eingesetzt. Aus anwendungstechnischer Sicht sind solche Alkyloligoglucoside bevorzugt, deren Oligomerisierungsgrad kleiner als 1,7 ist und insbesondere zwischen 1,2 und 1,4 liegt. Der Alkylrest R¹ kann sich von primären Alkoholen mit 16 bis 22 Kohlenstoffen ableiten. Typische Beispiele sind Alkyloligoglucoside auf Basis von Cetylalkohol, Stearylalkohol, Isostearylalkohol und/oder Behenylalkohol sowie deren technischen Gemischen. Vorzugsweise werden Alkyloligoglucoside eingesetzt, die sich von Fettalkoholen mit 16 bis 18 Kohlenstoffatomen ableiten, wie insbesondere dem Cetearylalkohol.

### Fettalkohole

Unter Fettalkoholen sind primäre aliphatische Alkohole der Formel **(II)** zu verstehen,

**R**^{**2**}**OH (II)**

in der R² für einen aliphatischen, linearen oder verzweigten Alkylrest mit 16 bis 22 Kohlenstoffatomen steht. Typische Beispiele sind Cetylalkohol, Stearylalkohol, Isostearylalkohol und Behenylalkohol sowie deren technische Mischungen, die z.B. bei der Hochdruckhydrierung von technischen Methylestern auf Basis von Fetten und Ölen anfallen. Bevorzugt sind technische Gemische mit 16 bis 18 Kohlenstoffatomen wie insbesondere Cetearylalkohol.

Im Sinne der Erfindung ist es besonders vorteilhaft, Mischungen von Alkyloligoglucosiden und Fettalkoholen einzusetzen, die identische Alkylreste aufweisen, also beispielsweise Mischungen von Cetearyloligoglucosiden und Cetearylalkohol.

### Kationpolymere

Geeignete kationische Polymere sind beispielsweise kationische Cellulosederivate, kationische Stärke, Copolymere von Diallylammoniumsalze und Acrylamide, quaternierte Vinylpyrrolidon/Vinylimidazol-Polymere wie z.B. Luviquat® (BASF AG, Ludwigshafen/FRG), Kondensationsprodukte von Polyglycolen und Aminen, quaternierte Proteinpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US sowie quaternierte Ammoniumsalz-Polymere wie z.B. Mirapol® A-15, Mirapol® AD-1 und Mirapol® AZ-1 der Miranol/US.

### Gewerbliche Anwendbarkeit

Die erfindungsgemäßen Emulsionen zeichnen sich auch bei Temperaturbelastung durch eine hohe Lagerstabilität sowie besonders vorteilhafte sensorische und avivierende Eigenschaften aus. In einer bevorzugten Ausführungsform der Erfindung enthalten die Emulsionen - bezogen auf den Emulgatoranteil - 20 bis 40 Gew.-% Alkyloligoglucoside, 60 bis 80 Gew.-% Fettalkohole und 1 bis 5 Gew.-% Kationpolymere. Der nichtwäßrige Anteil der Emulsionen, der sich weitgehend aus dem Emulgator- und dem Ölkörpergehalt zusammensetzt und dabei in der Regel dem Feststoffgehalt entspricht, liegt üblicherweise bei 5 bis 95 und vorzugsweise 15 bis 75 Gew.-%. Das bedeutet umgekehrt, daß die Emulsionen 5 bis 95 und vorzugsweise 25 bis 85 Gew.-% Wasser enthalten können, abhängig davon, ob Lotionen mit einer vergleichsweise niedrigen oder Cremes und Salben mit einer hohen Viskosität hergestellt werden sollen.

### Ölkörper

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht. Der Anteil der Ölkörper am nichtwäßrigen Anteil der Emulsionen kann 5 bis 99 und vorzugsweise 10 bis 75 Gew.-% ausmachen

### Hilfs- und Zusatzstoffe

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe Ölkörper, Emulgatoren, Überfettungsmittel, Fette, Wachse, Stabilisatoren, biogene Wirkstoffe, Glycerin, Konservierungsmittel, Farb- und Duftstoffe enthalten.

Als nichtionogene **O/W-Co-Emulgatoren** kommen in Betracht (a1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; (a2) C_{12/18}-Fettsäuremono- und -diester von Anlagerungsprodukten von 1 bis 30 Mol Ethylenoxid an Glycerin; (a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte; (a4) Anlage-rungsprodukte von 15 bis 60 Mol Ethylenoxid an Ricinusöl und/oder gehärtetes Ricinusöl; (a5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen. Die Anlagerungsprodukte von Ethylenoxid und/oder von Propylenoxid an Fettalkohole, Fettsäuren, Alkylphenole, Glycerin-mono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, ent-spricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Als **W/O-Co-Emulgatoren** kommen in Betracht: (b1) Anlagerungsprodukte von 2 bis 15 Mol Ethylenoxid an Ricinusöl und/oder gehärtetes Ricinusöl; (b2) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose); (b3) Trialkylphosphate; (b4) Wollwachsalkohole; (b5) Polysiloxan-Polyalkyl-Polyether-Copolymere bzw. entsprechende Derivate; (b6) Mischester aus Pentaerythrit, Fettsäuren, Citronensäure und Fettalkohol gemäß **DE-PS 11 65 574** sowie (b7) Polyalkylenglycole.

Der Anteil der Emulgatoren und Co-Emulgatoren am nicht-wäßrigen Anteil der Emulsionen kann 0,1 bis 10 und vorzugsweise 1 bis 7 Gew.-% betragen.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium und/oder Zinkstearat eingesetzt werden. Unter **biogenen Wirkstoffen** sind beispielsweise Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation "Kosmetische **Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt. Typische Emulsionen weisen Zusammensetzungen gemäß Tabelle 1 auf (mit der Maßgabe, daß sich die Angaben zu 100 Gew.-% ergänzen).

**Tabelle 1**

| **Typische Zusammensetzung von Lotionen und Cremes** | | |
|---|---|---|
| **Bestandteil** | **Lotion [Gew.-%]** | **Creme [Gew.-%]** |
| Alkylpolyglucoside | 1 - 2 | 1 - 3 |
| Fettalkohole | 2 - 3 | 2 - 6 |
| Kationpolymere | 0,05 - 0,2 | 0,05 - 0,2 |
| Ölkörper | 25 - 50 | 25 - 50 |
| Hilfs- und Zusatzstoffe | 1 - 5 | 1 - 5 |
| Wasser | 50 - 70 | 25 bis 50 |

Der Gesamtanteil der Hilfs- und Zusatzstoffe kann 1 bis 10, vorzugsweise 2 bis 5 Gew.-% - bezogen auf die Mittel - betragen. Die Herstellung der Mittel kann in an sich bekannter Weise, d.h. beispielsweise durch Heiß-, Heiß-Heiß/Kalt- bzw. PIT-Emulgierung erfolgen. Hierbei handelt es sich um ein rein mechanisches Verfahren, eine chemische Reaktion findet nicht statt.

### Beispiele

Es wurden Emulsionen hergestellt, enthaltend 35 Gew.-% Ölkörper (C1 bis C3), 5 Gew.-% Emulgator/Polymer- bzw. Emulgator/Tensid-Mischung und ad 100 Gew.-% Wasser. Die Herstellung der Emulsionen erfolgte nach der PIT-Methode, also oberhalb der Phaseninversionstemperatur. Die Rezepturen R1 und R2 enthalten die Kombination der Komponenten A1 bis A3 und sind erfindungsgemäß. Die Rezepturen R3 bis R8 enthalten zwar die Emulgatorkombination A1 und A2, jedoch ein nicht erfindungsgemäßes Tensid (B1 bis B6) und dienen zum Vergleich. Die Viskosität der Proben wurde nach der Brookfield-Methode in einem RVF-Viskosimeter (20 Upm, Spindel 1) sofort sowie nach Lagerung über 7 Tage bei 20 bzw. 40°C bestimmt. Die Ergebnisse sind in Tabelle 2 zusammengefaßt.

### Eingesetzte Substanzen (CTFA-Nomenklatur, soweit möglich)

A1) Hexadecyl Polyglucose
A2) Hexadecyl Alcohol
A3) Lauryldimonium hydroxypropyl hydrolyzed collagen
B1) Dodecylbenzolsulfonat-Natriumsalz
B2) Octadecensulfonat-Natriumsalz
B3) a-Sulfotalgfettsäuremethylester-Natriumsalz
B4) sekundäres Hexadecansulfonat-Natriumsalz
B5) Mono/Dilaurylphosphat-Mono/Di-Natriumsalz
B6) Cetylstearyldimethylammoniumbromid
C1) Dicapryl Ether
C2) Decyl Oleate
C3) Mandelöl

**Tabelle 2**

| **Viskosität und Lagerstabilität (Mengenangaben als Gew.-%)** | | | | | | | |
|---|---|---|---|---|---|---|---|
| **Komponenten** | **R1** | **R3** | **R4** | **R5** | **R6** | **R7** | **R8** |
| A1 | 1,9 | 1,9 | 1,9 | 1,9 | 1,9 | 1,9 | 1,9 |
| A2 | 3,0 | 2,85 | 2,85 | 2,85 | 2,85 | 2,85 | 2,85 |
| A3 | 0,1 | - | - | - | - | - | - |
| B1 | - | 0,5 | - | - | - | - | - |
| B2 | - | - | 0,5 | - | - | - | - |
| B3 | - | - | - | 0,5 | - | - | - |
| B4 | - | - | - | - | 0,5 | - | - |
| B5 | - | - | - | - | - | 0,5 | - |
| B6 | - | - | - | - | - | - | 0,5 |
| C1 | 15 | 15 | 15 | 15 | 15 | 15 | 15 |
| C2 | 10 | 10 | 10 | 10 | 10 | 10 | 10 |
| C3 | 5 | 5 | 5 | 5 | 5 | 5 | 5 |
| Wasser | ad 100 | | | | | | |

| ***Viskosität [mPas]*** | | | | | | | |
|---|---|---|---|---|---|---|---|
| ***- sofort*** | 9.800 | 10.000 | 10.000 | 12.000 | 11.000 | 10.000 | 8.000 |
| ***- nach 7 d, 20°C*** | 9.800 | 7.800 | 6.800 | 8.000 | 8.500 | 9.000 | 6.500 |
| ***- nach 7 d, 40°C*** | 9.500 | 4.500 | 3.000 | 2.500 | 2.000 | 1.500 | 1.000 |

Man erkennt, dass lagerstabile Emulsionen nur unter Verwendung der erfindungsgemäßen Dreierkombinationen, d.h. unter Einsatz der Kationpolymere erhalten werden.

## Patentansprüche

1. Kosmetische und/oder pharmazeutische Emulsionen, enthaltend - bezogen auf den Emulgatoranteil -
(a) mindestens 10 Gew.-% C₁₆-C₂₂-Alkyloligoglucoside,
(b) mindestens 50 Gew.-% C₁₆-C₂₂-Fettalkohole und
(c) 0,1 bis 10 Gew.-% kationische Polymere,
mit der Maßgabe, daß sich die Mengenangaben zu 100 Gew.-% ergänzen.

2. Emulsionen nach Anspruch 1, **dadurch gekennzeichnet, daß** sie Alkyloligoglucoside der Formel **(I)** enthalten,
**R**^{**1**}**O-[G]**_{**p**} **(I)**
in der R¹ für einen linearen, gesättigten Alkylrest mit 16 bis 22 Kohlenstoffatomen, G für einen Glucoserest und p für eine Zahl im Bereich von 1 bis 10 steht.

3. Emulsionen nach den Ansprüchen 1 und 2, **dadurch gekennzeichnet, daß** sie Fettalkohole der Formel **(II)** enthalten,
**R**^{**2**}**OH (II)**
in der R² für einen aliphatischen, linearen oder verzweigten Alkylrest mit 16 bis 22 Kohlenstoffatomen steht.

4. Emulsionen nach den Ansprüchen 1 bis 3, **dadurch gekennzeichnet, daß** sie kationísche Polymere enthalten, die ausgewählt sind aus der Gruppe, die gebildet wird von kationischen Cellulosederivaten, kationischen Stärken, Copolymeren von Diallylammoniumsalzen und Acrylamiden, quaternierten Vinylpyrrolidon/Vinylimidazol-Polymeren, Kondensationsprodukten von Polyglycolen und Aminen, quaternierten Proteinpolypeptiden, Polyethyleniminen, kationischen Siliconpolymeren, Copolymeren der Adipinsäure und Dimethylaminohydroxypropyldiethylentrimamin, Polyaminopolyamiden, kationischen Chitinderivaten, kationischem Guar-Gum und quaternierten Ammoniumsalz-Polymeren.

5. Emulsionen nach den Ansprüchen 1 bis 4, **dadurch gekennzeichnet, daß** sie Ölkörper enthalten, die ausgewählt sind aus der Gruppe, die gebildet wird von Guerbetalkoholem auf Basis von Fettalkoholen mit 6 bis 18 Kohlenstoffatomen, Estern von linearen C₆-C₂₀-Fettsäuren mit linearen C₆-C₂₀-Fettalkoholen, Estern von verzweigten C₆-C₁₃-Carbonsäuren mit linearen C₆-C₂₀-Fettalkoholen, Estern von linearen C₆-C₁₈-Fettsäuren mit verzweigten Alkoholen, Estern von linearen und/oder verzweigten Fettsäuren mit mehrwertigen Alkoholen und/oder Guerbetalkoholen, Triglyceriden auf Basis C₆-C₁₀-Fettsäuren, pflanzlichen Ölen, verzweigten primären Alkoholen, substituierten Cyclohexanen, Guerbetcarbonaten, Dialkylethern und/oder aliphatischen bzw. naphthenischen Kohlenwasserstorfen.

6. Emulsionen nach den Ansprüchen 1 bis 5, **dadurch gekennzeichnet, daß** sie einen nicht-wäßrigen Anteil von 5 bis 95 Gew.-% aufweisen.

7. Emulsionen nach den Ansprüchen 1 bis 6, **dadurch gekennzeichnet, daß** sie einen Emulgatoranteil von 0,1 bis 10 Gew.-% - bezogen auf den nicht-wäßrigen Anteil - aufweisen.

## Claims

1. Cosmetic and/or pharmaceutical emulsions containing - based on their emulsifier component -
(a) at least 10 % by weight of C₁₆₋₂₂ alkyl oligoglucosides,
(b) at least 50 % by weight of C₁₆₋₂₂ fatty alcohols and
(c) 0.1 to 10% by weight of cationic polymers,
with the proviso that the quantities add up to 100% by weight.

2. Emulsions as claimed in claim 1, **characterized in that** they contain alkyl oligoglucosides corresponding to formula **(I):**
**R**^{**1**}**O-[G]**_{**p**} **(I)**
in which R¹ is a linear saturated alkyl group containing 16 to 22 carbon atoms, G is a glucose unit and p is a number of 1 to 10.

3. Emulsions as claimed in claims 1 and 2, **characterized in that** they contain fatty alcohols corresponding to formula **(II):**
**R**^{**2**}**OH (II)**
in which R² is an aliphatic, linear or branched alkyl group containing 16 to 22 carbon atoms.

4. Emulsions as claimed in claims 1 to 3, **characterized in that** they contain cationic polymers selected from the group consisting of cationic cellulose derivatives, cationic starches, copolymers of diallyl ammonium salts and acrylamides, quatemized vinyl pyrrolidone/vinyl imidazole polymers, condensation products of polyglycols and amines, quatemized protein polypeptides, polyethyleneimines, cationic silicone polymers, copolymers of adipic acid and dimethylaminohydroxypropyl diethylenetriamine, polyaminopolyamides, cationic chitin derivatives, cationic guar gum and quatemized ammonium salt polymers.

5. Emulsions as claimed in claims 1 to 4, **characterized in that** they contain oil components selected from the group consisting of Guerbet alcohols based on fatty alcohols containing 6 to 18 carbon atoms, esters of linear C₆₋₂₀ fatty acids with linear C₆₋₂₀ fatty alcohols, esters of branched C₆₋₁₃ carboxylic acids with linear C₆₋₂₀ fatty alcohols, esters of linear C₆₋₁₈ fatty acids with branched alcohols, esters of linear and/or branched fatty acids with polyhydric alcohols and/or Guerbet alcohols, triglycerides based on C₆₋₁₀ fatty acids, vegetable oils, branched primary alcohols, substituted cyclohexanes, Guerbet carbonates, dialkyl ethers and/or aliphatic or naphthenic hydrocarbons.

6. Emulsions as claimed in claims 1 to 5, **characterized in that** they have a non-aqueous component of 5 to 95% by weight.

7. Emulsions as claimed in claims 1 to 6, **characterized in that** they have a percentage emulsifier content of 0.1 to 10% by weight, based on the non-aqueous component.

## Revendications

1. Emulsions cosmétiques et/ou pharmaceutiques contenant - rapporté à la proportion d'agent émulsionnant,
a) au moins 10 % en poids d'alkyle en C₁₆-C₂₂ oligoglucosides
b) au moins 50 % en poids d'alcools gras en C₁₆-C₂₂ et
c) de 0,1 à 10 % en poids de polymères cationiques avec la précision que les données en quantités se complètent à 100 % en poids.

2. Emulsions selon la revendication 1,
**caractérisées en ce qu'**
elles renferment des alkyloligoglucosides de formule (I)
R¹O-[G]ₚ (I)
dans laquelle R¹ représente un reste alkyle linéaire saturé ayant de 16 à 22 atomes de carbone, G représente un reste glucose, et p représente un nombre dans la zone de 1 à 10.

3. Emulsions selon les revendications 1 et 2,
**caractérisées en ce qu'**
elles renferment des alcools gras de formule II
R²OH (II)
dans laquelle R² représente un reste alkyle aliphatique, linéaire ou ramifié, ayant de 16 à 22 atomes de carbone.

4. Emulsions selon les revendications 1 à 3,
**caractérisées en ce qu'**
elles renferment des polymères cationiques qui sont choisis dans le groupe formé des dérivés de la cellulose cationiques, des amidons cationiques, des copolymères de sels de diallylammonium et d'acrylamides, des polymères de vinylpyrolidone/vinylimidazol quatermisés, des produits de condensation de polyglycols et d'amines, de polypetide protéine quaternisé, polyéthyléneimines, des polymères de silicone cationiques, des copolymères d'acide adipique et de diméthylaminohydroxypropyldiethylènetrimamine, des polyaminopolyamides, des dérivés cationique de la chitine de la gomme guar cationique et des polymères de sels d'ammonium quaternisés.

5. Emulsions selon les revendications 1 à 4,
**caractérisées en ce qu'**
elles renferment des composés huileux qui sont choisis dans le groupe formé des alcools de Guerbet à base d'alcools gras ayant de 6 à 18 atomes de carbone, des esters d'acides gras en C₆ - C₂₀ linéaires avec des alcools gras en C₆-C₂₀ linéaires, des esters d'acides en C₆-C₁₃ avec des alcools gras en C₆ - C₂₀ linéaires, des esters d'acides gras en C₆ - C₁₈ linéaires avec des alcools ramifiés, des esters d'acides gras linéaires et/ou ramifiés avec des alcools plurifonctionnels et/ou des alcools de Guerbet, des triglycérides à base d'acides gras en C₆-C₁₀, des huiles végétales, des alcools primaires ramifiés, des cyclohexanes substitués, des carbonates de Guerbet, des éther dialkyliques et/ou des hydrocarbures aliphatiques ou naphténiques.

6. Emulsions selon les revendications 1 à 5,
**caractérisées en ce qu'**
elles possèdent une proportion non aqueuse de 5 à 95 % en poids.

7. Emulsions selon les revendications 1 à 6,
**caractérisées en ce qu'**
elles possèdent une proportion d'agent émulsionnant allant de 0,1 à 10% en poids - rapporté à la fonction non aqueuse.
